# EUROPEAN PATENT APPLICATION

(11) **EP 2 267 118 A1**
(43) Date of publication of application: **29.12.2010**
(21) Application number: 09724127.7
(22) Date of filing: 27.03.2009
(51) Int. Cl.: C12N 5/10, A61K 35/14, A61K 48/00, A61P 35/00, A61P 37/04, C12N 15/09

(54) **METHOD FOR PRODUCTION OF TRANSFECTED CELL**

(30) Priority: 27.03.2008 JP 2008082453
(71) Applicant: Takara Bio, Inc., Otsu-shi Shiga 520-2193 (JP)
(72) Inventor: NUKAYA, Ikuei, Otsu-shi Shiga 520-2193 (JP); GOTO, Yumi, Otsu-shi Shiga 520-2193 (JP); TOSAKA, Yasuhiro, Otsu-shi Shiga 520-2193 (JP); MINENO, Junichi, Otsu-shi Shiga 520-2193 (JP); KATO, Ikunoshin, Otsu-shi Shiga 520-2193 (JP)
(74) Representative: Zwicker, Jörk
(86) International application number: PCT/JP2009/056264
(87) International publication number: WO 2009/119793

(57) **Abstract**

Disclosed is a method for producing a cell population into which a desired gene is introduced, which is **characterized by** comprising the following steps (1) and (2): (1) culturing a cell population containing a T-cell and/or a progenitor cell of a T-cell in a vessel containing fibronectin or a fragment thereof and a CD3 ligand; and (2) adding a vector carrying the desired gene to the vessel of the step (1). By carrying out both the steps (1) and (2) in the same vessel, the gene transfer can be achieved with high efficiency.

## Description

### Technical Field

The present invention relates to a method for producing a cell population containing a T-cell into which a desired gene is introduced, which is useful in the medical field.

### Background Art

Organisms are protected from several biological invasions mainly by immune responses, and an immune system consists of various cells and soluble factors they produce. Among them, white blood cell, in particular, lymphocyte plays a central role. This lymphocyte is divided into two major types, B lymphocyte (hereinafter also referred to as B-cell) and T lymphocyte (hereinafter also referred to as T-cell), both of which recognize antigens specifically and act on them to defend the organism.

In the periphery, CD4 T-cells having a CD (Cluster of Differentiation) 4 marker or CD8 T-cells having a CD8 marker occupy a large portion of T-cells. A large portion of CD4 T-cells is referred to as helper T-cell (hereinafter described as T_{H}), and is involved in assistance of antibody production and induction of various immune responses, and is differentiated by antigen stimulation into Th1 type or Th2 type, which respectively produce different kinds of cytokines. A large portion of CD8 T-cells is differentiated by antigen stimulation into cytotoxic T-cell [Tc: cytotoxic T lymphocyte; alias name: killer T-cell; hereinafter also referred to as CTL], which shows a cytotoxic activity.

Immunotherapy has been attracting interests as the fourth treatment for cancer, following surgical operation, chemotherapy and radiation therapy. As immunotherapy uses the immunity that a human originally has, physical stress on the patient is said to be less compared to other therapies. Therapies that transfer a lymphokine-activated cell, an NK T-cell, a γδ T-cell or the like, obtained by carrying out an expansion of CTL, a peripheral blood lymphocyte or the like which has been induced ex vivo according to various methods , dendritic cell transfer therapies, peptide vaccine therapies and Th1 cell therapy, by which an induction of antigen-specific CTL in vivo is expected, furthermore, immuno-gene therapies in which these cells are transduced ex vivo with genes that can be expected to have various effects and transferred into the body, and the like are known as immunotherapies.

A kind of cytotoxic T-cells (CTLs) can, through a specific T-cell receptor (hereinafter abbreviated as TCR), recognize a complex, which is a conjugate of a major histocompatibility complex molecule (MHC molecule, referred to as human leukocyte antigen in the case of human; hereinafter abbreviated as HLA) encoded by the major histocompatibility complex (hereinafter abbreviated as MHC) and an antigen peptide, and can kill a cell that presents this complex on the cell surface.

A cytotoxic activity specific to the target antigen can be conferred by introducing the gene of TCR that recognizes the target antigen into a T-cell such as CTL. Based on this finding, gene therapies with TCR gene are being attempted targeting various antigens, such as MART1 (Non-patent document 1), gp100 (Non-patent document 2) and mHAG HA-2 antigen (Non-patent document 3). In addition, gene therapies are being attempted using genes encoding TCR derived from human, TCR derived from an organism other than human, chimeric receptors of the antigen recognition site of the antibody that recognizes the target antigen, a group of molecules that associate with TCR to form an antigen recognition complex (for instance, CD3), a T-cell surface antigen (for instance, CD8 and CD28) and portions of these, as a receptor to be introduced into T-cell.

Fibronectin is a high-molecular weight glycoprotein that is present in the blood, on the cell surface and in the extracellular matrix of a tissue of an animal, having a molecular weight of 250,000, and is known to have multiple functions. In immunotherapies that transfer a lymphokine-activated cell obtained by expansion of CTL, a peripheral blood lymphocyte or the like, which had been obtained ex vivo through the stimulation of IL-2 and anti-CD3 antibody, the effects of fibronectin or a fragment thereof have been examined for problems such as how to maintain cytotoxic activity upon expansion of antigen-specific CTL induced ex vivo, and how to expand a lymphocyte ex vivo efficiently (for instance, Patent documents 1 to 3).

In recent years, it has been reported that, in immunotherapy, much higher therapeutic effect can be expected by administering, rather than a terminally differentiated effector T-cell, a naive T-cell or a central memory T-cell, which are in more undifferentiated states, to an organism (for instance Non-patent document 4 and 5). In addition, with the dendritic cell transfer therapy, the peptide vaccine therapy and the like, in which induction of antigen-specific CTL in vivo is expected, a sufficient effect can often not be obtained, for instance, in a patient with progressed cancer who is believed to have in vivo small population of naive T-cells, which are to become progenitors of CTL.
Patent document 1: WO 03/016511 pamphlet
Patent document 2: WO 03/080817 pamphlet
Patent document 3: WO 2005/019450 pamphlet
Non-patent document 1: Journal of Immunology, Volume 163, pp. 507-513 (1999)
Non-patent document 2: Journal of Immunology, Volume 170, pp. 2186-2194 (2003)
Non-patent document 3: Blood, Volume 103, pp. 3530-3540 (2003)
Non-patent document 4: Journal of Clinical Investigation, Volume 115, pp. 1616-1626 (2005)
Non-patent document 5: Journal of Immunology, Volume 175, pp. 739-748 (2005)

### Disclosure of Invention

### Problems to be Solved by the Invention

An object of the present invention is to provide a method for producing conveniently a cell population into which a desired gene is introduced, which is useful in the treatment of a disease by cell therapies, as well as a method for producing a cell population into which a desired gene is introduced with higher efficiency.

As a result of earnest studies to solve the above problems, the present inventors found that, with respect to a cell population cultured in a vessel containing fibronectin or a fragment thereof and a CD3 ligand, when the operation of introducing a vector carrying a desired gene is carried out in the same vessel, the gene transfer efficiency is increased compared to the prior art, and completed the present invention.

That is to say, the present invention relates to
[1] A method for producing a cell population into which a desired gene is introduced, comprising the following steps:
   (1) step of culturing a cell population containing a T-cell and/or a progenitor cell of a T-cell in a vessel containing fibronectin or a fragment thereof and a CD3 ligand; and
   (2) step of adding a vector carrying the desired gene to the vessel of step (1),
[2] the method according to [1], wherein, in step (2), the vector is a retrovirus vector,
[3] the method according to [1], wherein, in step (2), after addition of the vector, an operation of increasing physically the frequency of contact between the vector and the cell is carried out,
[4] the method according to [3], wherein, the frequency of contact between the vector and the cell is increased physically by adding of a centrifugal force or stirring of the content of the vessel,
[5] the method according to [1], further comprising the step of culturing the transduced cell population obtained in step (2),
[6] the method according to [1], further comprising the step of separating a desired sub-cell population from the transduced cell population obtained in step (2),
[7] a cell population into which a desired gene is introduced, which is obtainable by the method according to any of [1] to [6],
[8] a medicine comprising, as an active ingredient, a cell population into which a desired gene is introduced, which is obtainable by the method according to any of [1] to [6],
[9] a therapeutic or prophylactic method for a disease, comprising the step of administering to a subject an effective amount of the cell population into which a desired gene is introduced, which is obtainable by the method according to any of [1] to [6], and
[10] use of the cell population into which a desired gene is introduced, which is obtainable by the method according to any of [1] to [6], for preparing a medicine.

### Effect of the Invention

According to the producing method of the present invention, a population containing a high proportion of cells into which a desired gene is introduced is provided. The cell population obtainable by the producing method is extremely useful in the treatment of diseases by cell therapies.

### Brief Description of Drawings

Fig. 1 shows the efficiency of ZsGreen gene transfer; and
Fig. 2 shows the efficiency of AcGFP gene transfer.

### Best Mode for Carrying Out the Invention

In the present invention, "T-cell" means a cell, also referred to as T lymphocyte, which, among the lymphocytes that are involved in immune response, originates from the thymus gland. T-cells include helper T-cell, suppressor T-cell, cytotoxic T-cell, naive T-cell, memory T-cell, αβ T-cell, which expresses TCR consisting of an α chain and a β chain, and γδ T-cell, which expresses TCR consisting of a γ chain and a δ chain. In addition, a "progenitor cell of a T-cell", which has the capability of differentiating into a T-cell, can also be used in the present invention. Examples of "cell populations containing a T-cell and/or a progenitor cell of a T-cell" include peripheral blood mononuclear cell (PBMC), naive T-cell, memory T-cell, hematopoietic stem cell, umbilical cord blood mononuclear cell or the like. In addition, a variety of cell populations derived from hematopoietic cell containing a T-cell can be used in the present invention. These cells may have been activated by a cytokine such as IL-2 in vivo or ex vivo. As the these cells, those collected from an organism, or those obtained through in vitro cultivation, for instance, a T-cell population obtained by the method of the present invention can be used directly or after cryopreservation. In addition, for instance, a cell population obtained through a variety of inducing operation or separating operation from the cells used in the preparation of the T-cell population derived from an organism, for instance, any of a cell population obtained by separating CD8⁺ or CD4⁺ cell from cells such as PBMCs, can also be used. Note that, in the method of the present invention, for producing a cell population, materials containing the cells, for instance, blood such as peripheral blood and umbilical cord blood, blood from which constituents such as red blood cells or blood plasma has been removed, bone marrow solution or the like, can be used.

In the present invention, "fibronectin" and the fragment thereof may be obtained naturally or synthesized artificially, that is to say, non-natural. Fibronectin and the fragment thereof can be prepared, for instance, based on the disclosure by Ruoslahti E., et al. Journal of Biological Chemistry (J.Biol.Chem.), Volume 256, No. 14, pp7277-7281 (1981), from substance derived from natural origin, in a substantially pure form. Here, substantially pure fibronectin or fibronectin fragment described herein means that these do not essentially contain other proteins which present together with fibronectin naturally. The fibronectin and the fragment thereof can be used in the present invention respectively alone, or in a mixture form with several kinds of proteins. In addition, a single molecule of the fibronectin fragment or combination of plural fragments with different structures may be used.

Note that, while a number of splicing variants are known to exist for fibronectin, the fibronectin used in the present invention may be any variant as long as it shows the desired effects of the present invention. For instance, in a case of fibronectin derived from blood plasma, a region referred to as ED-B which presents in the upstream of the cell binding domain, and a region referred to as ED-A present between the cell binding domain and the heparin binding domain are known to be deleted; however, such fibronectin derived from blood plasma can also be used in the present invention.

Useful informational for the fibronectin fragment that can be used in the present invention, and the preparation of the fragment is available from Journal of Biochemistry (J. Biochem.), Volume 110, pp284-291 (1991), EMBO Journal (EMBO J.), Volume 4, No. 7, pp1755-1759 (1985), Biochemistry, Volume 25, No. 17, pp4936-4941 (1986) and the like. In addition, the nucleotide sequence encoding fibronectin and the amino acid sequence of fibronectin are disclosed in GenBank Accession No. NM_002026 and NP_002017.

The domain structure of fibronectin is divided into seven domains, in addition, three kinds of similar sequences are contained in the amino acid sequence thereof. The entirety is constituted with the repetition of each of these sequences. The three kinds of the similar sequences are referred to as Type I, Type II and Type III, respectively, among which Type III is constituted with 71 to 96 amino acid residues, and the matching rate of these amino acid residues is 17 to 40%. In fibronectin, 14 Type III sequences are present, among which, the 8th, 9th and 10th sequences (hereafter referred to as III-8, III-9 and III-10, respectively) are contained in the cell binding domain, and the 12th, 13th and 14th sequences (hereafter respectively referred to as III-12, III-13 and III-14) are contained in the heparin binding domain. In addition, a region referred to as IIICS is present on the C-terminal end of the heparin binding domain. A region comprising 25 amino acids having binding activity to VLA-4, referred to as CS-1, is present in IIICS. The fibronectin fragment that can be used in the present invention can be a fragment containing any domain of III-7, 8, 9, 11, 12, 13 and CS-1, and furthermore, may be a fragment in which plural domains are repeated and linked. For instance, a fragment containing the cell adhesion domain containing a ligand to VLA-5, the heparin binding domain, the CS-1 domain which is a ligand to VLA-4, or the like, is used in the present invention. For instance, CH-271, CH-296, H-271 and H-296 described in the above J. Biochem., Volume 110, pp284-291 (1991), and derivatives and variants thereof are indicated as examples of the fragments. The above CH-296 is commercially available under the name RetroNectin (registered trademark).

In the following, the present invention is described specifically.

### 1. Producing method of the present invention

The present invention relates to a method for producing a cell population into which a desired gene is introduced, comprising the following steps:
(1) step of culturing a cell population containing a T-cell in a vessel containing fibronectin or a fragment thereof and a CD3 ligand, and
(2) step of adding a vector carrying a desired gene to the vessel of step (1).
Note that the above step (1) may be described herein as the first step, and step (2) as the second step.

The cell population produced by the method of the present invention is a cell population containing a T-cell into which a desired gene is introduced. There is no particular limitation on the desired gene to be introduced, and it can be selected appropriately according to the purpose of introduction of the gene. Without any particular limitation, a gene encoding a polypeptide such as an enzyme, an antibody, a structural protein, a cytokine, a chemokine, a toxin or a fluorescent protein, a gene encoding an antisense nucleic acid, an RNA that results in RNA interference or the like can be introduced by the method of the present invention. In one aspect of the present invention, a gene encoding a receptor that recognizes the desired antigen is indicated as an example.

The above "receptor that recognizes the desired antigen" is a protein that recognizes the target antigen specifically. T-cell receptor (TCR) derived from human, TCR derived from an organism other than human and the like are indicated as examples of the receptors that recognize the desired antigen. As TCRs, known are a heterodimer consisting of α chain and β chain and a heterodimer consisting of γ chain and δ chain, either of which can be used appropriately in the present invention. In addition, a chimeric receptor in which the antigen recognition site of these TCRs or the antigen recognition site of an antibody that recognizes the desired antigen is combined with one or more components selected from a group of molecules that associates with TCR to form an antigen recognition complex (for instance, CD3ζ), T-cell surface antigens (for instance, CD8, CD28), and portions thereof (recombinant TCR) can also be used appropriately as a receptor that recognizes the desired antigen. The receptor may be a receptor constituted with one molecule, or a homodimeric or heterodimeric receptor constituted with plural molecules. As a receptor consisting of one molecule, a receptor having the antigen recognition site of scFv (single chain Fv) can also be used appropriately. Although not to be limited in particular, a receptor that recognizes the desired antigen may be constituted with an extracellular domain that recognizes the desired antigen, a transmembrane domain and an intracellular domain that transfers a signal, and may further contain a hinge or linker domain. In the present invention, the gene encoding a receptor that recognizes the desired antigen is an exogenous gene, and is a gene that is not present or expressed originally in T-cell into which the gene is introduced. That is to say, "a gene encoding a receptor that recognizes the desired antigen" means a gene that is introduced artificially into T-cell used in the present invention, including those derived from the same species as or different species from the T-cell. The cell population containing the T-cell transduced with the receptor gene is a cell population containing a T-cell that recognizes the desired antigen. The cell population is a population with high specificity to the antigen, compared to that into which no gene encoding the receptor is introduced, and is able to respond rapidly to stimulation of the desired antigen. In addition, in the producing method of the present invention, the desired antigens, well known proteins, cytokines, chemokines or other constituents may be added, in addition, by adding a separating or an isolating step, the cell population can be induced, cultured or isolated. Furthermore, the method for producing a cell population containing these cells is also included in the method of the present invention.

In the method of the present invention for producing the cell population, it is preferred that the total period of cultivation is 4 to 14 days. Note that "total period of cultivation" comprises that for the steps of (1) and (2), and, for instance, a step of cultivation carried out for the purpose of increasing the number of cells in addition to the above two steps. When the total period of cultivation is 4 to 14 days, the obtained cell population is a cell population with increased cell growth rate, increased proportion of naive T-like cells and increased level of IFN-γ production, and is suitable for use in the field of cell therapy. Note that, when the total period of cultivation is less than 4 days, the number of cells that is sufficient for use in common immunotherapies cannot be obtained. In the present invention, the total period of cultivation is more preferably 5 to 14 days and even more preferably 7 to 14 days.

In the method of the present invention for producing the cell population, the first step is a step in which a cell population containing a T-cell is cultured in a vessel containing fibronectin or a fragment thereof and a CD3 ligand. The present step is carried out for at least one day or more, more preferably 2 to 7 days, and even more preferably 2 to 5 days after cultivation initiation. Note that, in the present invention, the cultivation in the presence of the above active ingredients is not limited solely to this first step, but may be carried out for a cell into which a desired gene has been introduced, as necessary.

In the' present invention, there is no particular limitation on the concentration of fibronectin, a fragment thereof or mixtures thereof upon cultivation, but, for instance, 0.001 to 500 µg/mL, in particular, 0.01 to 500 µg/mL are preferred.

In the present invention, CD3 ligand is not limited in particular as long as it is a substance having the activity of binding to CD3, but, for instance, it may be an anti-CD3 antibody, particularly preferably an anti-CD3 monoclonal antibody may be used. For instance, OKT3 [Science, Volume 206, pp347-349 (1979)] is indicated as example. There is no particular limitation on the concentration of CD3 ligand in the culture medium, but, for instance, when using an anti-CD3 monoclonal antibody, for instance, 0.001 to 100 µg/mL, and in particular 0.01 to 100 µg/mL are preferred.

Additionally, in the present invention, cells can also be co-stimulated by adding other co-stimulating factors such as CD28 ligand, as necessary. The desired antigen, glucocorticoid-induced TNF-related receptor ligand (GITRL), anti-CD28 antibody, CD80, B7-1, B7-2 and the like are indicated as examples of co-stimulating factors.

The culture medium used in the method of the present invention for producing the cell population is not particularly limited as long as it contains the above active ingredients, but well known culture media prepared by mixing constituents that are necessary for T-cell expansion can be used. For instance, commercially available culture media can be selected and used appropriately. These culture media may contain cytokines, appropriate proteins and other constituents in addition to the original ingredients thereof. As cytokines, for instance, IL-2, IL-7, IL-12, IL-15, IFN-γ and the like are indicated as examples. Preferably, a culture medium containing IL-2 is used. There is no particular limitation on the concentration of IL-2 in the culture medium, but it may be for instance, preferably 0.01 to 1×10⁵ U/mL, more preferably 1 to 1×10⁴ U/mL. In addition, as an appropriate protein, for instance, anti-IL-4 antibody is indicated as example. Furthermore, a lymphocyte stimulating factor such as lectin can also be added. In addition, while the cell into which the desired gene is to be introduced may be activated by adding a γδ T-cell activating factor prior to transduction , it may also be activated after transduction. There is no particular limitation on the γδ T-cell activating factors as long as they show activating action and growth promoting action on γδ T-cell, but, for instance, they may be compounds of bisphosphonic acids such as pamidronate and zoledronate, pyrophosphate monoester compounds such as isopentenylpyrophosphate and 2-methyl-3-butenyl-1-pyrophosphate, phosphohalohydrins, phosphoepoxides or the like. There is no particular limitation on the concentrations of the constituents in the culture medium as long as the desired effects are obtained.

In addition, serum or blood plasma may be added into the culture medium. While there is no particular limitation on the amounts thereof added into the culture medium, 0% by volume to 20% by volume is given as examples in addition, the amount of serum or blood plasma used can be varied depending on the cultivation stage. For instance, decreasing the serum or plasma concentration in a stepwise manner is possible. Note that the origin of the serum or blood plasma may be autologous (meaning the origin is the same as the cultured cell) or non-autologous (meaning the origin is different from the cultured cell); however, from the point of view of safety, those from autologous origins are used appropriately.

Although there is no particular limitation on the number of cells at the initiation of cultivation in the present invention, it may be, for instance, preferably 10 cells/mL to 1×10⁸ cells/mL, more preferably 10² cells/mL to 5×10⁷ cells/mL, even more preferably 10³ cells/mL to 2×10⁷ cells/mL. In addition, there is no particular limitation on the culture condition, and the conventional conditions used for cell cultivation can be used. For instance, cultivation in conditions such as at 37°C and 5% CO₂ is possible herein. In addition, at an appropriate interval of time, diluting the cell culture solution by adding a fresh culture medium, replacing the culture medium, or replacing the cell culture instrument is possible.

There is no particular limitation on the cell culture instrument used in the producing method for the cell population of the present invention, but, for instance, plates, flasks, bags, large culture containers, bioreactors or the like can be used. Note that a CO₂ gas-permeable cell culture bag can be used as the bag. In addition, large culture containers can be used when large amounts of cell populations are prepared commercially. In addition, although the cultivation can be carried out in an open system or a closed system, it is preferred to carry out the cultivation in a closed system from the point of view of safety of the obtained cell population.

Note that, in addition to being dissolved to coexist in the culture medium, fibronectin or a fragment thereof and CD3 ligand, other co-stimulating factors, appropriate protein, cytokines or other constituents contained in the culture medium may also be immobilized on an appropriate solid phase, for instance, cell culture instrument (including both for an open system and a closed system) such as plates, flasks and bags, cell culture supports such as beads, membrane or slide glass. There is no particular limitation on the material of these solid phases as long as it can be used for cell cultivation. When the various constituents are immobilized on a culture instrument or cell culture support, preferably, the proportion of the amounts of the constituents to the amounts of culture medium used in the cultivation is adjusted to be the same proportion as the concentrations used when the constituents are dissolved in the culture medium; however, there is no limitation to this proportion as long as the desired effects are obtained.

There is no particular limitation on the method for immobilizing fibronectin or a fragment thereof, CD3 ligand, or other constituents onto a solid phase, but, for instance, these substances can be immobilized by contacting them with a solid phase in an adequate buffer solution.

In addition, regarding immobilization of fragment of fibronectin onto a solid phase, immobilization can also be carried out according to the methods described in WO 97/18318 pamphlet and WO 00/09168 pamphlet.

When the various constituents described above or the active ingredient used in the present invention on a solid phase are immobilized, the active ingredients and the like can be trapped by merely separating the T-cell population from the solid phase after culturing a T-cell population according to the method of the present invention, which can prevent the active ingredients and the like from mixing into the T-cell population.

In the method of the present invention for producing the cell population, the second step is a step in which a vector carrying the desired gene is added to the vessel of the first step, to introduce the gene into a cell. That is to say, the first step and the second step are carried out in the same vessel, without exchanging the vessel. Addition of the vector is not limited to once, and the vector may be added several times during the second step. Addition of the vector can be carried out by an appropriate way, such as, addition of a solution containing the vector to the culture solution in the vessel, or replacing the culture medium in the vessel with a culture medium containing the vector.

Though there is no limitation on the number of desired genes to be introduced into a cell, it may be one gene, or a plurality of genes including several genes. For instance, according to the cell to be transduced, a suitable gene such as a gene encoding a T-cell surface antigen can be introduced simultaneously, beforehand, or subsequently. For instance, in case αβ TCR gene into a γδ T-cell is introduced, it is preferred to introduce, in addition to the gene, a gene encoding CD8.

In the present invention, there is no particular limitation on the vector carrying the desired gene, but, it can be selected from well known vectors and used appropriately. For instance, either of a method using a virus vector and a method using a non-virus vector may be used in the present invention. Various documents have been published for the details of these methods.

Though there is not any particular limitation on the above virus vector and well known virus vectors normally used in transduction method can be used, for instance, retrovirus vector (including lentivirus vector, pseudo-typed vector), adenovirus vector, adeno-associated virus vector, simian virus vector, vaccinia virus vector, sendai virus vector or the like can be used. Preferably, a retrovirus vector, an adenovirus vector or a lentivirus vector can be used. As the virus vector, one lacking replication capability, in which self-replication in the infected cell is inhibited, is suitable. In addition, a substance for improving the gene transfer efficiency, such as RetroNectin (registered trademark, manufactured by Takara Bio Inc.) can also be used upon transduction.

Although there is no limitation on the non-virus vector used in the present invention, for instance, a plasmid vector can be used as a non-virus vector, and introduced by a method using a carrier such as liposome, ligand-polylysine or the like, or the calcium phosphate method, the electroporation method, or the particle gun method or the like.

In the present invention, the desired gene is introduced in a cell in the presence of fibronectin or a fragment thereof and a CD3 ligand. The fibronectin or the fragment thereof used in the present invention has the action increasing efficiency of gene transfer with a retrovirus vector or a lentivirus vector. In addition, a retrovirus vector and a lentivirus vector can introduce an exogenous gene in the vector stably in the chromosome DNA of the cell into which the vector is to be introduced, and are used for the purpose of gene therapy or the like. Because the vectors may infect cells in division or growth, they are particularly appropriate to carry out transduction in the step of the producing method of the present invention.

For instance, the desired gene can be inserted into a vector, a plasmid or the like and used so as to express the gene under the control of a suitable promoter. In addition, in order to achieve efficient transcription of the gene, another regulatory element that cooperates with the promoter or transcription initiation site, for instance, an enhancer sequence and/or a terminator sequence may be present in the vector. In addition, for the purpose of insertion by homologous recombination into the chromosome of the T-cell targeted for transduction, for instance, the gene may be placed between flanking sequences comprising nucleotide sequences respectively homologous to the nucleotide sequences present on both ends of the desired target insertion site of a gene in the chromosome.

While the second step may be carried out after a given period has passed from the beginning of the first step, it may also be carried out simultaneously to the beginning of the first step. In the latter case, a cell population containing a T-cell and a vector carrying the desired gene are added simultaneously to a vessel containing fibronectin or a fragment thereof and a CD3 ligand. In a preferred aspect of the present invention, but not to limit the present invention, the second step is carried out at least one day or longer after carrying out the first step, more preferably 1 to 7 days, and even more preferably 2 to 5 days. The second step, but not to limit the present invention, is carried out in general for 1 to 3 days and preferably for 1 to 2 days after carrying out the first step.

In addition, for the purpose of increasing the contact frequency between the cell and the virus in the second step, an operation that physically increases the contact frequency between the vector and the cell may be carried out after addition of the vector. For instance, the contact frequency between the vector and the cell can be increased by adding a centrifugal force to the vessel to move the vector and the cell to the bottom surface of the vessel and by stirring the contents of the vessel. Stirring of the contents can be carried out preferably by vibrating or shaking the vessel. The gene transfer efficiency into the cell is further improved by these operations. As a preferred aspect, addition of centrifugal force to the vessel containing the vector is indicated as an example. Though the centrifugal force to be added is not limited in particular as long as it is in a range by which the cell does not receive excessive damage and the gene transfer efficiency improves, in general, 250 to 2000xg, and preferably 500 to 1500×g is preferred. In addition, 3 to 120 minutes and in particular 5 to 60 minutes are appropriate durations for adding centrifugal force.

The method of the present invention is characterized by cultivation of the cell population containing the T-cell to be transduced in a vessel containing fibronectin or a fragment thereof and a CD3 ligand followed by carrying out transduction into the cell population in the same vessel. Conventionally, a pre-culture of cells was carried out in the presence of an appropriate stimulating factor and then, the cells were transferred to another vessel to carry out transduction. According to the present invention, loss or unnecessary stimulation of cells due to the transfer can be avoided, and the efficiency of gene transfer can be improved. In particular, the method of the present invention is appropriate for transduction using a culture vessel of a closed system, such as a cell culture bag.

The method of the present invention for producing the cell population may further comprises as a third step, the step of culturing the transduced cell obtained in the second step. That is to say, the vector may be removed after finishing the second step (for instance, by replacing the culture medium with that without the vector), and cultivation of the cell can be continued.

The above step can be carried out by the conventional method of cell cultivation, for instance, a well known cultivation method for T-cells. The cultivation of cells may be carried out in the same conditions as the above first step, or in a similar condition to those used in the first step except for the use of fibronectin or a fragment thereof and a CD3 ligand used as active ingredients in the above method.

In addition, the producing method of the present invention can also comprise a step for separating the cell population into desired sub-cell populations, before or after each step. For example, as describe above, a high proportion of naive T-like cells is contained in a cell population obtained by cultivation in the presence of the active ingredient of the present invention. Therefore, by further separating and obtaining a cell expressing the desired surface antigen marker, naive T-like cells or a T-cell population containing naive T-like cells at higher rates can be obtained. In addition, cell expressing the desired gene may be separated and obtained after the second step of the producing method of the present invention. Although there is no particular limitation on separating operation, separation can be carried out by well known methods using, for instance, a cell sorter, magnetic beads, columns or the like. In addition, when there is an appropriate means, a sub-cell population containing a cell into which a receptor gene is introduced may be selected.

In addition, by cloning T-cell from the cell population produced by the method of the present invention, it is also possible to maintain a stable T-cell. In addition, the cell population obtained by the method of the present invention can also be used for additional cultivation by the method of the present invention or well known method to obtain a new cell population.

The cell population obtained by the method of the present invention contains a cell expressing CD45RA and expressing CD62L or CCR7, that is to say, a naive T-like cell, which is an undifferentiated T-cell, in a high rate. While the cytotoxic activity of the cell population can also be evaluated by a well known in vitro test, the cell population obtained by the producing method of the present invention does not necessarily demonstrate high cytotoxic activity in such evaluation systems since the T-cell population obtained by the present invention as described above contains undifferentiated naive T-like cells at a high rate.

In addition, compared to a cell population prepared by the conventional methods, the cell population produced by the method of the present invention has excellent viability in the organism, retaining in the organism a high activity against an antigen of the same species or different species. Accordingly, the method of the present invention is a novel T-cell expanding method for adoptive immunotherapy comprising the steps of stimulating the cell with fibronectin or a fragment thereof and introducing a desired gene, and the transduced T-cell obtained by the method of the present invention is effectively transplanted into an organism and maintained. That is to say, the method of the present invention can provide a cell population for use in cell therapy, which can persist at high concentration in the organism over a long period and is more useful, and can be broadly applied to all the gene therapy methods based on T-cell.

### 2. Cell population, medicine, therapeutic method or prophylactic method and use of the present invention

The cell population produced by the method of the present invention is a cell population that may show the desired effect depending on the introduced gene. In addition, compared to a cell population prepared by the conventional method, the proportion retaining the desired gene is higher. For instance, a cell population into which a gene encoding a receptor that recognizes the desired antigen is introduced, is useful for the treatment of a variety of diseases because it demonstrates a cytotoxic activity against a cell presenting an antigen recognized by the receptor encoded by the introduced receptor gene. Although there is no particular limitation on the disease for which the cell population is administered, for instance, cancers (leukemia, solid tumors and the like), hepatitis, infectious diseases caused by a virus (influenza virus, HIV or the like), a bacteria (Mycobacterium tuberculosis, MRSA, VRE or the like), a fungus (Aspergillus, Candida, Cryptococcus and the like) are indicated as examples. In addition, the cell population produced by the method of the present invention can also be used for the prevention of infectious disease after a bone marrow transplantation or irradiation, donor lymphocyte infusion for the purpose of remission of relapsed leukemia, or the like.

Furthermore, the present invention provides a pharmacutical composition (therapeutic agent) comprising the above cell population as an active ingredient. The therapeutic agent containing the cell population is appropriate for use in immunotherapy. In an immunotherapy, a T-cell appropriate for the treatment in a patient is administered, for instance, via injection or drip infusion transvenously, transarterially, subdermally, intraperitoneally or the like. The therapeutic agent is extremely useful in uses for the above-mentioned diseases or donor lymphocyte infusion. The therapeutic agent can be prepared, for instance, as a drip infusion agent or an injectable according to a well known method in the pharmaceutical field, by mixing the T-cell population prepared by the method of the present invention as an active ingredient, with a well known organic or inorganic carrier, diluent, stabilizer or the like, which is appropriate for parenteral administration. Note that the content of cell population of the present invention in the therapeutic agent, the dosage of the therapeutic agent and the conditions for the therapeutic agent can be determined appropriately according to well known immunotherapies. For instance, although there is no particular limitation on the content of T-cell population of the present invention in a medicine, for instance, it may be preferably 1×10³ to 1×10¹¹ cells/mL, more preferably 1×10⁴ to 1×10¹⁰ cells/mL and even more preferably 1×10⁵ to 1×10⁹ cells/mL. In addition, although there is no particular limitation on the dosage of the medicine of the present invention, for instance, it may be for adult preferably 1×10⁶ to 1×10¹² cells/day, more preferably 1×10⁷ to 5×10¹¹ cells/day and even more preferably 1×10⁸ to 2×10¹¹ cells/day. In addition, combinations of the immunotherapy with the therapeutic agent and a medicine therapy by administration of well known medicines, or a treatment by radiotherapy or surgical operation can be used.

In addition, the present invention provides a diagnostic agent comprising the cell population as an active ingredient. For example, by analyzing and screening the cells obtained from a patient with the diagnostic agent of the present invention, inference on the therapeutic effects, selection of an effective transgene and the like are allowed.

The present invention further provides a therapeutic method or prophylactic method for a disease comprising administering to a subject an effective amount of the cell population obtained by the above method. Although there is no particular limitation on the subject herein, preferably, an organism (for instance human patient, or non-human animal) with the above-mentioned disease for which the T-cell population prepared by the method of the present invention is administered, is indicated. Note that the therapeutic agent of the present invention containing as an active ingredient a cell population into which a gene encoding the T-cell receptor is introduced, is administered to a subject expressing an HLA molecule that is identical to or has up to three locus mismatches with the HLA molecule expressed by the cell population.

In addition, the effective amount herein is the amount of the T-cell population, exerting therapeutic or prophylactic effects when the T-cell population is administered to the subject, compared to a subject into which the T-cell population has not been administered. While a specific effective amount can vary depending on the dosage form, administrating method, purpose of use, age and body weight of the subject, symptoms and the like, preferably, it is similar to the above-mentioned medicines. The administrating method is also not limited, but, for instance, administration by drip infusion, injection or the like is preferred, similarly to the above medicines.

In addition, the present invention can produce a cell population containing an activated T-cell by providing the cell population obtained by the producing method of the present invention described above with a stimulation of at least one stimulating factor selected from the group consisting of a cell having the capability of presenting an antigen, a cell presenting an antigen, an antigen, a CD3 ligand, a CD28 ligand, a cytokine, a chemokine or a cell having the capability of producing a cytokine. Furthermore the present invention provides a cell population obtained by the above producing method. The cell population containing an activated T-cell obtained in the above can be used as an active ingredient of a pharmaceutical composition, similarly to the T-cell population obtained by the above producing method. Herein, there is no particular limitation on the stimulation of the stimulating factor as long as it is one in which the above-mentioned cell population obtained by the producing method of the present invention is activated by the stimulating factor, but, for instance, stimulation may be provided by carrying out cultivation under the simultaneous presence of the T-cell population obtained by the producing method of the present invention and the stimulating factor.

In the present specification, there is no particular limitation on a cell having the capability of presenting an antigen as long as it is a cell generally used as an antigen-presenting cell, but, for instance, dendritic cell, γδ T-cell, monocyte, B-cell, T-cell, macrophage, fibroblast, Langerhans' cell, and cell population containing at least one of the above cells, and particularly preferably, dendritic cell, γδ T-cell, T-cell, B-cell, monocyte, macrophage, and cell population containing at least one of the above cells are indicated as examples. In addition, while the origin of the cell having the capability of presenting an antigen may be autologous or non-autologous for the patient to be administered, autologous origin is preferred. Herein, cell having the capability of presenting an antigen means a cell having the capability of presenting an antigen, but not a cell presenting an antigen.

In the present specification, as a cell presenting an antigen, any of a cell having the capability of presenting the above antigen, to which an appropriate antigen has been added artificially, a cell in which a gene is introduced to express the antigen, or a cell collected from an organism, which has been presenting an antigen, can be used.

In the present specification, there is no particular limitation on a presented antigen as long as it allows a peptide to be presented on an antigen-presenting cell or is recognized by a T-cell, allowing the T-cell to be activated efficiently, but for instance, peptides, glycopeptides, tumor cell extracts, tumor cell sonicates and tumor cell hot water extracts, nucleic acids (DNA and RNA) from a virus or the like, bacteria, proteins and the like are indicated as examples.

In addition, herein, the mentioned CD3 ligands and CD28 ligands are indicated as examples of CD3 ligand and CD28 ligand. Note that by providing co-stimulation of anti-CD3 antibody and anti-CD28 antibody to the cell population obtained by the producing method of the present invention, an activated lymphocyte population having cytokine production capability can be prepared.

In the present specification, a cytokine is not limited in particular as long as it can act on and activate a T-cell, however, for instance, IL-2, IFN-γ, TGF-β, IL-15, IL-7, IFN-α, IL-12, CD40L, IL-27 and the like are indicated as examples, and from the point of view of enhancing cellular immunity, IL-2, IFN-γ and IL-12 are particularly preferably indicated as examples.

In the present specification, there is no particular limitation on a chemokine as long as it acts on a T-cell and demonstrates migration activity, however, for instance, RANTES, CCL21, MIP1α, MIP1β, CCL19, CXCL12, IP-10 and MIG are indicated as examples.

In the present specification, there is no particular limitation on a cell having the capability of producing a cytokine as long as it is a cell with the capability of producing the cytokines, but, for instance, from the point of view of enhancing cellular immunity, Th1 cell is indicated as an example.

By adding antigen stimulation to a T-cell population obtained by the producing method of the present invention, induction of a useful antigen-specific CTL is allowed, with extremely high cytotoxic activity and high antigen recognition capability. Furthermore, in addition to the above-mentioned stimulating factors, cultivation can be carried out in the presence of the above-mentioned fibronectin, fragment thereof or mixtures thereof or known constituents used in cultivation of a T-cell. The T-cell population produced in the present invention can survive in an organism over a long period, being an extremely useful T-cell population having high therapeutic effects.

Note that in the present specification, production of cell or cell population is synonymous to cultivation of cell or cell population, and means a process that comprises each step of induction (activation), maintenance, expansion of the cell or cell population, or steps combining these.

### Example

Hereafter, the present invention will be described more specifically by giving examples; however, the present invention is not to be limited only to the following examples.

In addition, among the operations described in the present specification, basic operations were according to methods described in Molecular Cloning: A Laboratory Manual 3rd ed., compiled by T. Maniatis et al., issued by Cold Spring Harbor Laboratory, 2001.

Example 1: Preparation of transduced cell with ZsGreen expressing retrovirus vector

### (1) Preparation of virus vector

A ZsGreen expressing retrovirus vector was prepared as follows: First, PCR was carried out with pZsGreen (manufactured by Clontech) as template, using the A2 primer with the nucleotide sequence of SEQ ID:1 and the ZsGreen primer with the nucleotide sequence of SEQ ID:2 to obtain an amplification fragment containing the region encoding ZsGreen. This was inserted into the downstream of the TCR receptor β subunit gene of the human T-cell receptor expression vector MS-bPa described in the document (Gene Therapy, February 21st, 2008, online edition (PMID: 18288212) to obtain pMS-abZ.

### (2) Preparation of producer cell

An ecotropic retrovirus was prepared from a 293 T-cell transformed with the plasmids pGP and pE-eco contained in the Retrovirus Packaging Kit (manufactured by Takara Bio Inc., Shiga, Japan) as well as pMS-abZ. After producer cell PG13 (ATCC#: CRL-10686) was infected three times with the obtained ecotropic retrovirus, clones were selected by limiting dilution to obtain an MS-abZ-producing cell.

### (3) Preparation of virus solution

After removing the supernatant from the culture of MS-abZ-producing cell line and washing the sample once with 5 mL of PBS (phosphate-buffered saline), MS-abZ-producing cells were treated with 1.5 mL of Trypsin/EDTA at room temperature for one minute and suspended in DMEM containing 10% fetal bovine serum and 50 units/mL penicillin-50 µg/mL streptomycin. Inoculation was carried out at 5×10⁶ cells per one φ100mm dish (manufactured by Iwaki). After cultivation for 24 hours the culture supernatant was removed, 8 mL of fresh culture medium containing 5mM sodium butyrate was added to the dish. Cultivation was further carried out for 24 hours, and the supernatant was recovered, filtered with a 0.45 µm filter and stored at -80°C. This supernatant was used as the MS-abZ virus solution in the subsequent experiments.

### (4) Preparation of ZsGreen transduced cell

Dissolved in PBS was a fibronectin fragment (RetroNectin, manufactured by Takara Bio Inc.) so as to be 25 µg/mL and an anti-CD3 antibody (OKT3, Janssen Pharmaceutical K.K.) so as to be 5 µg/mL. This solution was added at 0.4 mL/well to a tissue culture treated 12-well plate and allowed to stand at room temperature for 5 hours. Thereafter, the solution was removed, and PBS was used to wash the plate twice with 1 mL/well, then, by adding 1 mL/well of RPMI1640 and washing the plate, and an fibronectin fragment/anti-CD3 antibody-immobilized plate was obtained. In addition, as a positive control for the transduction method, a plate for cells used in the method of re-plating cells on another transduction virus binding plate upon transduction (RN Binding method), was prepared as follows. The fibronectin fragment/anti-CD3 solution was added at 1.2 mL/well to a tissue culture-treated 6-well plate and allowed to stand at room temperature for 5 hours. Thereafter, the solution was removed, and PBS was used to wash the plate twice with 2 mL/well, then, by adding 2 mL/well of RPMI1640 to wash the plate, and an fibronectin fragment/anti-CD3 antibody-immobilized plate was obtained.

A plate for use in the RN Binding method was prepared as follows:
A fibronectin fragment was dissolved in PBS so as to be 20 µg/mL, and this solution was added to a tissue culture-untreated 12-well plate at 1 mL/well and allowed to stand overnight at 4°C. After removing the solution, PBS (2 mL/well) was used to wash the plate twice, 3 mL of a two-fold diluted MS-abZ virus solution was added to the well, and centrifugation was carried out at 32°C, 2000xg for 2 hours. After centrifugation, the virus solution was removed, PBS containing 1.5% human serum albumin (HSA) (3 mL/well) was used to wash the plate twice to prepare the virus binding plate.

A Human peripheral blood mononuclear cell (PBMC) was suspended so as to be 0.3×10⁶ cells/mL in GT-T503 culture medium (manufactured by Takara Bio Inc.) containing 1% autologous blood plasma, 600 IU/mL IL-2, 0.2% HSA and 2.5 µg/mL Fungizone, and this suspension was added to the fibronectin fragment/anti-CD3 antibody-immobilized plate so as to obtain 0.75 mL/cm² to initiate the cultivation.

A transducing operation was carried out as follows. After cells were plated on the fibronectin fragment/anti-CD3 antibody-immobilized plate, MS-abZ virus solution was added to each well so as to be diluted two-fold on day 0, day 1 and day 2, allowing to stand or to be centrifuged at 1000xg for one hour, and further cultured as-is until day 3. On day 3, the cells were recovered, and prepared to obtain 2×10⁵ cells/mL, and 2 mL of this cell suspension was re-plated on a 24-well plate and cultured in a CO₂ incubator.

By contrast, in the RN Binding method, PBMCs cultured for 3 days in the presence of fibronectin fragment/anti-CD3 antibody were prepared to obtain respectively 1×10⁶/cm² and 2×10⁶ cells/mL, and 2 mL of this cell suspension was added to the virus binding plate. After the plate was centrifuged at 32°C, 1000×g for 10 minutes, cultivation was carried out for 4 hours at 37°C in a CO₂ incubator. The cultured cells were prepared so as to obtain 2×10⁵ cells/mL, and 2 mL of this cell suspension was plated on a 24-well plate and cultured in a CO₂ incubator.

The cultured cells were recovered on day 7 from the initiation of PBMC cultivation, and the gene transfer efficiency was measured as the proportion of ZsGreen-positive cells.

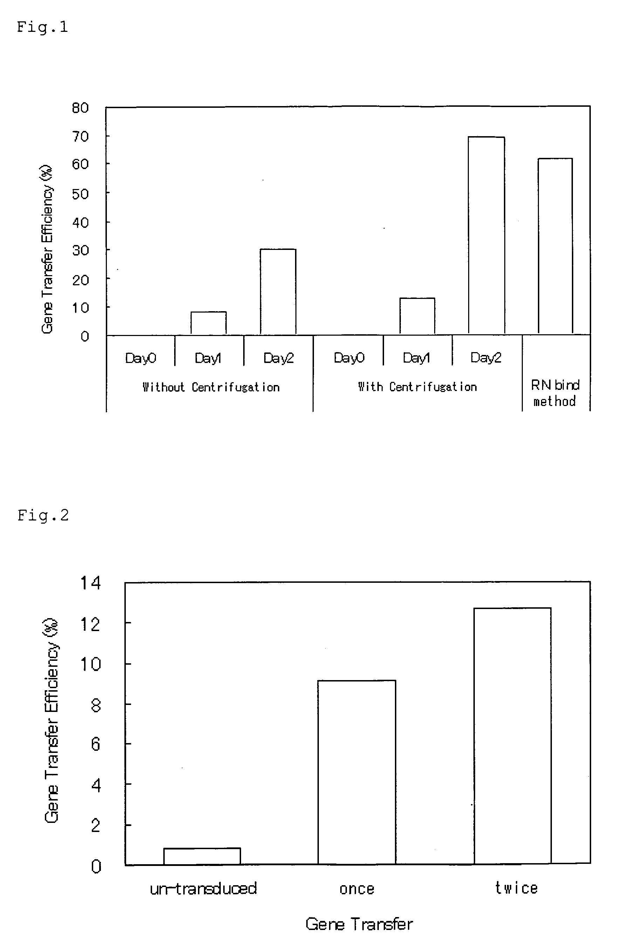

The measured results of gene transfer efficiency are shown in Fig. 1. As to the gene transfer efficiency, an increase was observed in the sample to which the virus solution was added on day 1 from the initiation of the cultivation, and there was more increases in the sample into which the solution was added on day 2. In addition, the gene transfer efficiency was further increased when a centrifugation operation was added after addition of the virus solution.

### Example 2: Effects of repeated transduction (1) Preparation of AcGFP expression virus vector

AcGFP expression vector MT-AcGFP was prepared as follows: First, PCR was carried out with pIRES2-AcGFP1 (manufactured by Clontech) as template, using AcGFP5 primer with the nucleotide sequence of SEQ ID:3 and AcGFP3 primer with the nucleotide sequence of SEQ ID:4 to obtain an amplification fragment. This was inserted into a MluI-BglII site of pMT vector [pM vector described in Gene Therapy Volume 7, pp797 - 804 (2000)] to prepare pMT-AcGFP.

### (2) Preparation of producer cell

An ecotropic retrovirus was prepared from a 293 T-cell transformed with the plasmids pGP and pE-eco contained in the Retrovirus Packaging Kit (manufactured by Takara Bio Inc., Shiga, Japan), and pMT-AcGFP. After producer cell PG13 (ATCC#: CRL-10686) was infected three times with the obtained ecotropic retrovirus, several clones were obtained by limiting dilution. Clones were selected based on the results of measuring the amounts of viral RNA in the culture supernatant by real-time PCR, and the results of measuring the amounts of AcGFP expression for the cultured cells brought into contact with the supernatant, serving as indicators, to obtain MT-AcGFP-producing cells.

### (3) Preparation of virus solution

AcGFP expressing retrovirus was prepared with a similar method to those of Example 1 (3). The obtained supernatant was used as MT-AcGFP virus solution in the subsequent experiments.

### (4) Preparation of AcGFP transduced cell

As described in Example 1 (4), a fibronectin fragment/anti-CD3 antibody solution was added to a tissue culture-treated 6-well plate at 1.2 mL/well, and allowed to stand at room temperature for 5 hours. Thereafter, the solution was removed, and PBS was used to wash the plate twice (2 mL/well), then, by adding 2 mL/well of RPMI1640 to wash the plate, and a fibronectin fragment/anti-CD3 antibody immobilized plate was obtained.

PBMC was suspended so as to obtain 0.3×10⁶ cells/mL in a GT-T503 culture medium (manufactured by Takara Bio Inc.) containing 1% autologous blood plasma, 600 IU/mL IL-2, 0.2% HSA and 2.5 µg/mL Fungizone, and this suspension was added to the fibronectin fragment/anti-CD3 antibody immobilized plate so as to obtain 0.75 mL/cm².

### (5) transducing operation

After plating cells on an fibronectin fragment/anti-CD3 antibody immobilized plate, MT-AcGFP virus solution was added to the wells so as to be diluted two-fold on day 2, centrifuged at 1000xg for one hour, and cultured until day 3. In addition, in the condition of carrying out a second transducing operation, a similar operation to that of day 3 was repeated. On day 3, in case transducing operation was carried out once or twice, cells were recovered on day 3 or day 4, respectively, and preprared to obtain 2×10⁵ cells/mL, and 2 mL thereof was re-plated on a 24-well plate and cultured in a CO₂ incubator.

On day 8 from the initiation of PBMC cultivation, the cultured cells were recovered, and the gene transfer efficiency was measued as the proportion of AcGFP-positive cell.

The measured results of gene transfer efficiency are shown in Fig. 2. As to AcGFP positive rate, in case of the fibronectin fragment/anti-CD3 antibody immobilized plate, by repeating virus addition into the same plate, an increase in the gene transfer efficiency was observed.

### Example 3: Preparation of transduced cells by using a retrovirus vector

Dissolved in PBS were a fibronectin fragment CH-296 [RetroNectin (registered trademark), manufactured by Takara Bio Inc.] so as to obtain 25 µg/mL, and an anti-CD3 antibody (OKT3, Janssen Pharmaceutical K.K.) so as to obtain 5 µg/mL. This solution was added at 0.4 mL/well to a tissue culture treated 12-well plate, and allowed to stand at room temperature for 5 hours. Thereafter, the solution was removed, and the wells were washed twice with 0.5 mL of PBS, and then, washed with 0.5 mL of RPMI1640 culture medium to obtain an CH-296/anti-CD3 antibody immobilized plate. A Human peripheral blood mononuclear cell (PBMC) were suspended so as to obtain 0.3×10⁶ cells/mL in a GT-T503 culture medium (manufactured by Takara Bio Inc.) containing 1% autologous blood plasma, 600 IU/mL IL-2, 0.2% HSA and 2.5 µg/mL Fungizone (hereafter represented by culture medium), and 3 mL of the cell suspension was added to an CH-296/anti-CD3 antibody immobilized 12-well plate, and cultivation was carried out under the condition of 37 °C and 5% CO₂.

As Control 1, PBMC was suspended so as to obtain 0.3×10⁶ cells/mL in the culture medium containing 30ng/mL OKT3, and 3 mL of cell suspension was added to a 12-well plate, and cultivation was carried out under the condition of 37°C and 5% CO₂.
As Control 2, OKT3 was immobilized at a concentration of 5 µg/mL on a tissue culture-treated 12-well plate, and PBMC was suspended so as to obtain 0.3×10⁶ cells/mL in the culture medium, and cultivation was carried out under the condition of 37°C and 5% CO₂.
As Control 3, suspended in the culture medium were PBMC so as to obtain 0.3×10⁶ cells/mL, and anti-CD3 antibody/anti-CD28 antibody-solid-phased beads (Dynabeads M450 CD3/CD28 T-cell Expander, manufactured by Invitrogen) so as to obtain 0.3×10⁶ cells/mL, and 3 mL of the cell suspension was added to a 12-well plate, and cultivation was carried out under the condition of 37°C and 5% CO₂.

On day 2 from the initiation of the cultivation, 1.5 mL of supernatant was removed from each well, and 1.5 mL of the MT-acGFP virus solution prepared in Example 2 was added, and transduction was carried out under the conditions of 32°C, 1000×g for one hour, and diluting the cell culture solution was carried out on day 3 and day 7, and gene transfer efficiency was analyzed as the proportion of ZsGreen-positive cells with a flow cytometer on day 7. In addition, on day 10, cells were recovered, the number of cells was counted by trypan blue staining, and the expansion rates for the 10-day cultivation was calculated from the dilution ratios on day 3 and day 7. The measured results of gene transfer efficiency are shown in Table 1, and the cell growth rates are shown in Table 2, respectively.

**Table 1: Gene transfer efficiencies under each condition**

| | Gene transfer efficiency (%) |
|---|---|
| CH-296/OKT3 | 24.7 |
| Control 1 | 2.7 |
| Control 2 | 1.9 |
| Control 3 | 3.3 |

**Table 2: Cell growth rates under each condition**

| | Growth rate (fold) |
|---|---|
| CH-296/OKT3 | 12.7 |
| Control 1 | 5.2 |
| Control 2 | 5.3 |
| Control 3 | 6.3 |

As shown in Table 1, it is demonstrated that efficient transduction into PBMC is allowed by using an CH-296/OKT3 immobilized vessel upon stimulation cultivation of PBMC and transduction with a retrovirus vector on day 2. As shown in Table 2, it is revealed that by carrying out stimulation cultivation in an CH-296/OKT3 immobilized vessel, a greater number of cells can be obtained compared with by OKT3 alone or co-stimulation by CD3/CD28 antibody.

### Example 4: Preparation of lentivirus vector

The lentivirus vector pLenti6.3/V5-TOPO (manufactured by Invitrogen) was blunted with DNA Blunting Kit (manufactured by Takara Bio Inc.), circularized with DNA Ligation kit Mighty Mix (manufactured by Takara Bio Inc.), and then transfected into One Shot Stbl3 (manufactured by Invitrogen) to prepare the lentivirus vector plasmid pLenti6.3/V5.
The plasmid pZsGreenN1 (manufactured by Clontech) was digested with the restriction enzyme NotI, then, blunted with DNA Blunting Kit, and thereafter, digested with the restriction enzyme BamHI to obtain the region encoding the green fluorescent protein ZsGreen. The lentivirus vector plasmid pLenti6.3/V5 was digested with the restriction enzyme XhoI, then blunted with the DNA Blunting Kit, and thereafter digested with the restriction enzyme BamHI. Using the DNA Ligation kit Mighty Mix, the ZsGreen gene fragment obtained above was inserted in the restriction enzyme-digested pLenti6.3/V5, and transfected into the One Shot Stbl3 to construct the ZsGreen expressing lentivirus vector plasmid pLenti6.3-ZsGreen.

The 293T/17 cell (ATCC CRL-11268) for preparing the lentivirus vector was cultured in DMEM culture medium containing 10% fetal bovine serum (FBS). To prepare the VSV-G pseudo-type lentivirus, ViraPower^{™} Packaging Mix (manufactured by Invitrogen), in which the VSV-G envelope expression plasmid and a gag-pol expression plasmid, which is a structural gene of lentivirus and a Rev expression plasmid, which is an accessory gene of lentivirus are mixed, and the ZsGreen expressing lentivirus vector plasmid pLenti6.3-ZsGreen constructed above were transduced into 293T/17 cell using TransIT 293 (manufactured by Mirus). The culture medium was replaced with a fresh one after 24 hours, and cultivation was continued. On day 2 from the initiation of the transduction, the culture supernatant was collected, and filtered with a 0.45 µm filter, and this filtrate was used as virus supernatant in the subsequent experiments.

### Example 5: Preparation of transduced cells by using a lentivirus vector

An CH-296/anti-CD3 antibody immobilized plate was prepared by the method described in Example 3. PBMC was suspended so as to obtain 0.7×10⁶ cells/mL in a culture medium, and 0.4 mL of cell suspension and 1 mL of lentivirus vector prepared in Example 4 were mixed, and added to an CH-296/anti-CD3 antibody immobilized 24-well plate, and centrifugal treatment at 32°C, 1000xg for 30 minutes was carried out, and whereafter a cultivation was carried out under the conditions of 37°C and 5% CO₂.

As Control 4, suspended in culture medium were PBMC so as to obtain 2×10⁶ cells/mL, and anti-CD3 antibody/anti-CD28 antibody solid-phased beads (Dynabeads M450 CD3/CD28 T-cell Expander, manufactured by Invitrogen) so as to obtain 4×10⁶ cells/mL, and incubated at room temperature for 30 minutes. 0.5 mL of cell suspension and 1 mL of the lentivirus vector prepared in Example 4 were mixed, and added to an CH-296 immobilized 24-well plate, and centrifugal treatment at 32°C, 1000xg for 2 hours was carried out, and whereafter 0.5 mL of culture medium was added to carry out cultivation under the conditions of 37°C and 5% CO₂.
As Control 5, 0.5 mL of the above cell suspension and 1 mL of the lentivirus vector prepared in Example 4 were mixed, and added to a tissue culture-untreated 24-well plate, and protamine was further added so as to obtain 10 µg/mL, and centrifugal treatment at 32°C, 1000×g for 2 hours was carried out, and whereafter 0.5 mL of culture medium was added to carry out a cultivation under the conditions of 37°C and 5% CO₂.

Diluting the cell culture solution was carried out on day 4, day 7 and day 10 from the initiation of the cultivation, and cells were recovered on day 14, and the gene transfer efficiency was analyzed as the proportion of ZsGreen-positive cell with a flow cytometer. In addition, the number of cells was counted by trypan blue staining, and the expansion rate in the culture on day 14 was calculated from the dilution ratios on day 4, day 7 and day 10. In addition, surface markers of the cells on day 14 were analyzed using CD4, CD8, CD45RA and CCR7 antibodies. The measured results of the gene transfer efficiency are shown in Table 3, and the cell growth rates in Table 4, and the results of cell surface marker analysis in Table 5 and Table 6, respectively.

**Table 3: Gene transfer efficiencies under each condition**

| | Gene transfer efficiency (%) |
|---|---|
| CH-296/OKT3 | 68.3 |
| Control 4 | 30.9 |
| Control 5 | 34.9 |

**Table 4: Cell growth rates in each condition**

| | Growth rate (fold) |
|---|---|
| CH-296/OKT3 | 257.8 |
| Control 4 | 27.2 |
| Control 5 | 17.9 |

**Table 5: Immunophenotype analysis (CD4/8)**

| | CD4 positive rate (%) | CD8 positive rate (%) |
|---|---|---|
| CH-296/OKT3 | 12.1 | 87.9 |
| Control 4 | 33.9 | 66.1 |
| Control 5 | 37.3 | 62.7 |

**Table 6: Immunophenotype analysis (Naive, Memory)**

| | Naïve (%) | Central Memory (%) | Effecter Memory (%) | Terminal Differentiated (%) |
|---|---|---|---|---|
| CH-296/OKT3 | 42.7 | 4.0 | 9.8 | 43.6 |
| Control 4 | 24.8 | 17.1 | 25.7 | 32.4 |
| Control 5 | 31.0 | 21.6 | 19.0 | 28.4 |

As shown in Table 3, it is demonstrated that efficient transduction into PBMC is allowed by using an CH-296/OKT3 immobilized vessel upon stimulation cultivation of PBMC and transducing the cell with a lentivirus vector. As shown in Table 4, it is revealed that by carrying out stimulation cultivation in an CH-296/OKT3 immobilized vessel, significantly greater number of cells, compared with co-stimulation with CD3/CD28 antibody, can be obtained. In addition, it is revealed from the analysis of cell surface markers that, by using an CH-296/OKT3 immobilized vessel, compared with co-stimulation by CD3/CD28 antibody, there are more CD8-positive killer cells, and the proportion of naive cells is also high.

### Industrial Applicability

According to the producing method of the present invention, a cell population into which a desired gene has been introduced with high efficiency is provided. The producing method of the present invention has a convenient effect, improving working efficiency. The cell population obtainable according to the producing method can be used appropriately in, for instance, immunotherapies. Consequently, the method of the present invention is expected to contribute greatly in the medical field.

### Sequence listing free text

SEQ ID NO: 1: Synthetic primer A2 to amplify a DNA fragment encoding ZsGreen
SEQ ID NO: 2: Synthetic primer ZsGreen to amplify a DNA fragment encoding ZsGreen
SEQ ID NO: 3: Synthetic primer AcGFP5 to amplify a DNA fragment encoding AcGFP
SEQ ID NO: 4: Synthetic primer AcGFP3 to amplify a DNA fragment encoding AcGFP

## Claims

1. A method for producing a cell population into which a desired gene is introduced, comprising the following steps:
(1) step of culturing a cell population containing a T-cell and/or a progenitor cell of a T-cell in a vessel containing fibronectin or a fragment thereof and a CD3 ligand; and
(2) step of adding a vector carrying the desired gene to the vessel of step (1).

2. The method according to claim 1, wherein, in step (2), the vector is a retrovirus vector.

3. The method according to claim 1, wherein, in step (2), after addition of the vector, an operation for increasing physically the frequency of contact between the vector and the cell is carried out.

4. The method according to claim 3, wherein the frequency of contact between the vector and the cell is increased physically by addition of a centrifugal force or stirring of the content of the vessel.

5. The method according to claim 1, further comprising the step of culturing the transduced cell population obtained in step (2).

6. The method according to claim 1, further comprising the step of separating a desired sub-cell population from the transduced cell population obtained in step (2).

7. A cell population into which a desired gene is introduced, which is obtainable by the method according to any one of claims 1 to 6.

8. A medicine comprising, as an active ingredient, a cell population into which a desired gene is introduced, which is obtainable by the method according to any one of claims 1 to 6.

9. A therapeutic or prophylactic method for a disease, comprising the step of administering to a subject an effective amount of the cell population into which a desired gene is introduced, which is obtainable by the method according to any one of claims 1 to 6.

10. Use of the cell population into which a desired gene is introduced, which is obtainable by the method according to any one of claims 1 to 6, for producing a medicine.


SEQUENCE LISTING

<110>  TAKARA BIO INC.

<120>  Method for producing gene-transduced cells

<130>  38-126

<140>  EP 09 724 127.7
<141>  2009-03-27

<150>  JP 2008-082453
<151>  2008-03-27

<160>  4

<170>  PatentIn version 3.3

<210>  1
<211>  58
<212>  DNA
<213>  Artificial Sequence

<220>
<223>  Synthetic primer A2 to amplify a DNA fragment encoding ZsGreen

<400>  1
aagtctgcta acatgcggtg acgtcgagga gaatcctggc cccatggccc agtccaag       58


<210>  2
<211>  21
<212>  DNA
<213>  Artificial Sequence

<220>
<223>  Synthetic primer ZsGreen to amplify a DNA fragment encoding
       ZsGreen

<400>  2
ctcgagtcag ggcaaggcgg a                                               21


<210>  3
<211>  30
<212>  DNA
<213>  Artificial Sequence

<220>
<223>  Synthetic primer AcGFP5 to amplify a DNA fragment encoding AcGFP

<400>  3
acgcgtagat ctatggtgag caagggcgcc                                      30


<210>  4
<211>  21
<212>  DNA
<213>  Artificial Sequence

<220>
<223>  Synthetic primer AcGFP3 to amplify a DNA fragment encoding AcGFP

<400>  4
ggatcctcac ttgtacagct c                                               21